(19) Europäisches Patentamt
European Patent Office
Office européen des brevets

(11) Veröffentlichungsnummer : **0 332 124 B1**

(12) # EUROPÄISCHE PATENTSCHRIFT

(45) Veröffentlichungstag der Patentschrift : **21.08.91 Patentblatt 91/34**

(51) Int. Cl.[5] : **C07C 45/46**, C07C 49/813, C07C 49/84, C07C 317/22, C07C 323/09

(21) Anmeldenummer : **89103951.3**

(22) Anmeldetag : **07.03.89**

(54) **Verfahren zur Herstellung von halogenhaltigen aromatischen Verbindungen.**

(30) Priorität : **09.03.88 DE 3807623**

(43) Veröffentlichungstag der Anmeldung : **13.09.89 Patentblatt 89/37**

(45) Bekanntmachung des Hinweises auf die Patenterteilung : **21.08.91 Patentblatt 91/34**

(84) Benannte Vertragsstaaten : **BE CH DE FR GB IT LI NL SE**

(56) Entgegenhaltungen :
**EP-A- 0 057 503**
**EP-A- 0 075 390**
**FR-A- 2 147 318**

(73) Patentinhaber : **HOECHST AKTIENGESELLSCHAFT**
**Postfach 80 03 20**
**W-6230 Frankfurt am Main 80 (DE)**

(72) Erfinder : **Hackenbruch, Joachim, Dr.**
**Berliner Strasse 20**
**W-6095 Ginsheim-Gustavsburg (DE)**
Erfinder : **Papenfuhs, Theodor, Dr.**
**Heinrich-Bleicher-Strasse 40**
**W-6000 Frankfurt am Main 50 (DE)**
Erfinder : **Warning, Klaus, Dr.**
**Kreuzheck 6**
**W-6239 Eppstein/Taunus (DE)**
Erfinder : **Siegemund, Günter, Dr.**
**Frankfurter Strasse 21**
**W-6238 Hofheim am Taunus (DE)**

EP 0 332 124 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren zur Herstellung von halogenhaltigen aromatischen Verbindungen, die mindestens zwei aromatische Ringe enthalten und über wenigstens ein Brückenglied miteinander verbunden sind.

Aus der GB-PS 1139296 ist die Herstellung von 1,4-Bis-(4-chlorbenzoyl)-benzol bekannt. Dabei werden 700 Teile (6,22 mol) Chlorbenzol mit 97 Teilen (0,48 mol) Terephthalsäuredichlorid und 145 Teilen (1,09 mol) gepulvertem wasserfreiem Aluminiumchlorid in 6 Stunden bei 130°C umgesetzt. Eine Ausbeute wird nicht angegeben. Die analoge Umsetzung von Fluorbenzol ist u.a. in der DE-OS 3531837 beschrieben, wobei das Halogenbenzol (hier Fluorbenzol), Aluminiumchlorid und Terephthalsäuredichlorid in praktisch den gleichen Molverhältnissen angewandt werden wie nach der GB-PS 1139296. Allerdings ist die Ausbeuteangabe für das 1,4-Bis-(4-fluorbenzoyl)-benzol in der DE-OS 3531837 zweifelhaft, da nach der Reinigung mehr Produkt erhalten wird (1250 g) als Rohprodukt (1225 g) vorhanden war. Auch ist der Schmelzpunkt mit 121°C (statt 218,5 bis 219,5°C gemäß Hergenrother et al. in Journ. Pol. Sci., Teil A, Polymer Chemistry, 25, 1094 (1987)) falsch angegeben.

Es ist ein Nachteil bisher bekannter Verfahren, daß eine große Menge an Aluminiumtrichlorid eingesetzt werden mußte, das bei der Aufarbeitung mit dem Abwasser abgeführt werden mußte und dieses belastete. Gleiches gilt für die übrigen in der DE-OS 3531837 genannten Lewis-Säuren Eisenchlorid, Titantetrachlorid und Zinntetrachlorid. Es ist daher wünschenswert, solche Verfahren zu entwickeln, bei denen die Katalysatorsysteme in unterstöchiometrischen Mengen verwendet, leicht aufgearbeitet und zurückgewonnen werden können. Einen Schritt in diese Richtung beschreiben Effenberger et al. in der DE-OS 2139994 mit der Herstellung von aromatischen Ketonen in Gegenwart von weniger als der molaren Menge Perfluoralkansulfonsäuren, wobei in allen 8 Beispielen Monoketone in guten Ausbeuten hergestellt werden. Nachteilig ist dabei aber, daß das Reaktionsgemisch schon nach einmaligem Einsatz zur Rückgewinnung des Katalysators mit Wasser behandelt werden muß.

Gegenstand der vorliegenden Erfindung ist nun ein Verfahren zur Herstellung von halogenhaltigen aromatischen Verbindungen der Formel (I) (siehe Patentanspruch 1), worin Hal Fluor, Chlor oder Brom und Z eine aromatische Gruppierung bedeuten, das dadurch gekennzeichnet ist, daß man ein Halogenbenzol der Formel $C_6H_5Hal$ (II) unter wasserfreien Bedingungen mit einem Bissäurehalogenid der Formel Hal-CO-Z-CO-Hal (III) im Molverhältnis von mindestens 2 : 1 in Gegenwart von 15 bis 500 mol-%, bezogen auf das Bissäurehalogenid (III), an Halogenalkansulfonsäuren der Formel $Y(C_nX_{2n})SO_3H$ (IV), vorzugsweise der Formel $Y(C_nF_{2n})SO_3H$ (V) umsetzt, wobei in den Formeln (II) und (III) Hal und Z die zuvor angegebene Bedeutung haben und Y Fluor oder Wasserstoff, X Fluor und/oder Chlor und n eine ganze Zahl von 1 bis 10 bedeuten, wobei mindestens ein X für Fluor steht.

Bevorzugt ist die Herstellung von Verbindungen (I), die durch wenigstens eines der Merkmale gekennzeichnet sind, daß Hal Fluor oder Chlor und Z Phenylen, zweckmäßig in anderer als ortho-Stellung, insbesondere p-Phenylen ist und daß Halogen in einer anderen als der o-Stellung zur CO-Gruppe steht. Verbindungen mit p-ständigem Halogen sind ganz besonders bevorzugt.

Als Halogenbenzol der Formel (II) kommt z.B. Brombenzol in Frage. Bevorzugt sind aber Fluorbenzol und Chlorbenzol. Das Halogenbenzol wird bevorzugt im Überschuß verwendet, so daß es als Lösungsmittel für das Bissäurehalogenid (III) dient. Im allgemeinen werden je Mol Bissäurehalogenid (III) 6 bis 40, vorzugsweise 10 bis 30 Mol Halogenbenzol (II) eingesetzt.

In den Bissäurehalogeniden III ist Z ein Phenylenrest oder z.B. der Rest $C_6H_4$-E-$C_6H_4$ mit E = O, $(CG_2)_m$, CO, S, SO, $SO_2$ oder $Si(CH_3)_2$, worin G Wasserstoff, Methyl, Fluor oder Trifluormethyl und m eine ganze Zahl von 0 bis 4 ist. Wenn m 0 ist, sind die beiden Phenylenreste also durch eine einfache Bindung verbunden.

Als Beispiele für Bissäurehalogenide seien genannt: Terephthalsäuredichlorid, Isophthalsäuredichlorid, 1,4- und 2,6-Naphthalindicarbonsäuredichlorid, 1,5-Anthracendicarbonsäuredichlorid, 4,4'-Biphenyldicarbonsäuredichlorid, Bis-(4,4'-chlorcarbonylphenyl)-methan, 2,2-Bis-(4,4'-chlorcarbonylphenyl)-propan, Hexafluor-2,2-bis-(4,4'-chlorcarbonylphenyl)-propan, Di-(4,4'-chlorcarbonylphenyl)-keton, Di-(4,4'-chlorcarbonylphenyl)-äther, Di-(4,4'-chlorocarbonylphenyl)-sulfid, Di-(4,4'-chlorcarbonylphenyl)-sulfoxid, Di-(4,4'-chlorcarbonylphenyl)-sulfon. Bevorzugt sind Terephthalsäuredichlorid, Di-(4,4'-chlorcarbonylphenyl)-keton, 4,4'-Biphenyldicarbonsäuredichlorid, Di-(4,4'-chlorcarbonylphenyl)-äther, Di-(4,4'-chlorcarbonylphenyl-sulfid, Di-(4,4'-chlorcarbonylphenyl)-sulfon.

Die als Beispiele genannten Verbindungen der Formel II und der Formel III sind entweder bekannt oder nach analogen Verfahren herstellbar.

Als Endprodukte der Formel I erhält man beispielsweise 1,4-Bis-(4-fluorbenzoyl)-benzol, 1,4-Bis-(4-chlorbenzoyl)-benzol, 4,4'-Bis-(4-fluorbenzoyl)-diphenyläther, 4,4'-Bis-(4-fluorbenzoyl)-biphenyl, 4,4'-Bis-(4-fluorbenzoyl)-diphenylsulfid und 4,4'-Bis-(4-fluorbenzoyl)-diphenylsulfon.

Das erfindungsgemäße Verfahren wird im allgemeinen bei Temperaturen von 20 bis 200°C, vorzugsweise von 60 bis 180°C und vorteilhaft auch unter sauerstofffreien Bedingungen durchgeführt. Man kann bei Atmosphärendruck arbeiten, aber auch bei leichtem Überdruck, insbesondere bis zum Eigendruck des Systems, der insbesondere durch die angewandte Temperatur, aber auch durch andere Faktoren, wie das Ausmaß der Füllung des Reaktionsgefäßes bestimmt wird. Beispielsweise kann er im Bereich bis zu 10 oder 15 bar liegen. Wenn man bei erhöhtem Druck arbeitet, kann man das Verfahren beispielsweise in einem Autoklaven aus Edelstahl, der gegebenenfalls mit einem resistenten Material wie Polytetrafluoräthylen ausgekleidet ist, durchführen. Durch die Anwendung des Überdrucks kann die Reaktion beschleunigt werden, jedoch kann damit auch eine erhöhte Bildung von Nebenprodukten verbunden sein.

Es ist auch möglich, Lösungsmittel mitzuverwenden, die unter den Reaktionsbedingungen gegenüber den Reaktionsteilnehmern inert sind, z.B. aliphatische Kohlenwasserstoffe oder Halogenderivate davon. Gewöhnlich sind hiermit aber keine Vorteile verbunden.

Ein Vorteil des erfindungsgemäßen Verfahrens besteht darin, daß die nach der Abtrennung der ausgefallenen Bis-(4-halobenzoyl)-verbindung erhaltene Mutterlauge wieder mit den Bissäurehalogeniden III und frischem Halogenbenzol versetzt und so als Reaktionsmedium vielfach, z.B. bis zu 10 Mal, wiederverwendet werden kann, ohne daß merkliche Ausbeuteverluste eintreten. Bei zu häufiger Rückführung, z.B. nach der zehnten Rückführung, wird eine Anreicherung von Nebenprodukten, insbesondere partiellen Umsetzungsprodukten, beobachtet, so daß das Reaktionsgemisch dann aufgearbeitet werden muß, wobei der Katalysator nach üblichen Verfahren, z.B. gemäß der DE-OS 2139994 (= GB-PS 1378913), zurückgewonnen werden kann. Die Möglichkeit, die Mutterlauge mehrfach zu nutzen, läßt sich natürlich auch so verwirklichen, daß man nach jedem Ansatz einen kleinen Teil, z.B. 5 bis 20%, ausschleust und durch neues Lösungsmittel und Katalysator ersetzt.

Die erfindungsgemäß verwendeten Katalysatoren der Formel IV enthalten vorzugsweise nicht mehr als 3 Chloratome, insbesondere höchstens ein Chloratom. Die Gruppe $C_nX_{2n}$ bzw. $C_nF_{2n}$ kann geradkettig oder verzweigt sein, wobei Verbindungen, in denen Y Wasserstoff ist, diesen vielfach in β-Stellung enthalten.

Die erfindungsgemäß verwendeten perfluorierten Katalysatoren sind bekannt (vgl. DE-OS 2139994), ebenso die β-H-Perfluoralkansulfonsäuren (J. Am. Chem. Soc. 75, 4595-4596 (1953). Soweit sie noch nicht bekannt sind, können sie nach üblichen Verfahren erhalten werden. Werden sie in zu geringen Mengen, nämlich in dem in der DE-OS 2139994 in den Beispielen angewandten Mengenbereich von 0,01 bis 10 mol-%, bei dem erfindungsgemäßen Verfahren eingesetzt, so werden die gewünschten Bis-(4-halobenzoyl)-verbindungen nicht oder nur in geringen Ausbeuten, oder zu stark verunreinigt, erhalten, wie durch die Vergleichsversuche V1 und V2 belegt wird. Geeignete Verbindungen der Formel IV sind z.B. Perfluor-n-octansulfonsäure, Perfluorhexansulfonsäure, Perfluorbutansulfonsäure, Pentafluoräthansulfonsäure, β-H-Perfluorheptansulfonsäure und β-H-Perfluorpentansulfonsäure. Naturgemäß können auch Mischungen verschiedener Katalysatoren verwendet werden. Bevorzugt sind Trifluormethansulfonsäure, 2-Chlor-1,1,2-trifluoräthansulfonsäure, 2-Hydroperfluoräthansulfonsäure und/oder 2-Hydroperfluorpropansulfonsäure. Vorzugsweise setzt man den Katalysator im ersten Ansatz in Mengen von 50 bis 150 mol-%, bezogen auf das Bissäurehalogenid III, zu. Verwendet man die Mutterlauge nach Abtrennung des erwünschten Reaktionsproduktes wieder, indem man sie zurückführt, braucht kein weiterer Katalysator zugesetzt zu werden, so daß im Ergebnis in der Regel eine weitaus geringere Menge an Katalysator verwendet wird als z.B. der halben stöchiometrischen Menge entspricht.

Die nach dem erfindungsgemäßen Verfahren erhaltenen Bis-(4-halobenzoyl)-verbindungen, von denen das 1,4-Bis-(4-fluorbenzoyl)-benzol und 1,4-Bis-(4-chlorbenzoyl)-benzol bevorzugt sind, sind wichtige Monomere für chemikalienbeständige Kunststoffe, die auch bei hohen Temperaturen noch beständig sind. Sie können nach Auflösen in geeigneten Lösungsmitteln, gegebenenfalls unter Zusatz fester Basen, vorzugsweise $Na_2CO_3$, $K_2CO_3$ oder MgO, und eventuell nachfolgende Filtration ohne weitere Reinigung direkt für Polykondensationen verwendet werden, z.B. mit Hydrochinon zu Polyätherketonen.

Die Erfindung wird durch die nachfolgenden Beispiele erläutert. Darin bedeutet h Stunde und BFB 1,4-Bis-(4-fluorbenzoyl)-benzol.

**Beispiele :**

1) Bei Raumtemperatur wurden 1730 g (18 mol) Fluorbenzol, 203 g (1 mol) Terephthalsäuredichlorid und 150 g (1 mol) Trifluormethansulfonsäure vorgelegt und 20 h unter Rückfluß (Kp 85°C) erhitzt. Das Reaktionsgemisch wurde bis zum Erkalten gerührt, das ausgefallene 1,4-Bis-(4-fluorbenzoyl)-benzol (BFB) abfiltriert und mit 192 g (2 mol) Fluorbenzol gewaschen. Die den Katalysator enthaltende Mutterlauge und die Waschlösung wurden vereint, mit 203 g Terephthalsäuredichlorid versetzt und die Reaktion in der angegebenen Weise wiederholt. Die Mutterlauge konnte zehn Mal rückgeführt werden, ohne daß eine merkliche Ausbeuteverminderung festzustellen war.

Zur Bestimmung des Reingehaltes wurde eine kleine Menge des durch anhaftendes Fluorbenzol feuchten Rohproduktes getrocknet und mittels Hochdruckflüssigkeitschromatographie untersucht. Die Ausbeute, bezogen auf Dicarbonsäurehalogenid, betrug nach zehn Rückführungen 95,1% d.Th. ; Fp. 218-221°C.

Das Produkt ließ sich wie folgt weiterverarbeiten : 450 g des feuchten BFB wurden mit 600 g Diphenylsulfon (DPS) und 10 g MgO langsam erhitzt. Nach Abdestillation des Fluorbenzols wurde eine Stunde auf 200°C erhitzt und das Reaktionsgemisch einer Klärfiltration unterworfen. Das so als Diphenylsulfon-Lösung erhaltene BFB konnte ohne weitere Reinigung direkt einer Polykondensation unterworfen werden. Die Ausbeute, bezogen auf BFB, betrug 90%, die Reinheit (ohne DPS) : über 99%.

1820 g der nach der zehnten Rückführung verbleibenden Mutterlauge wurden mit 500 ml Wasser versetzt; es wurde vom ausgefallenen Feststoff abgetrennt, die beiden flüssigen Phasen getrennt, die wäßrige Phase mit 61,5 g (1,1 mol) KOH versetzt, das Wasser am Rotationsverdampfer abgetrennt, der Rückstand mit 200 g $H_2SO_4$ (98,4%ig) versetzt und die Trifluormethansulfonsäure bei Atmosphärendruck abdestilliert. Es wurden 92 g (= 61% d.Th.) Trifluormethansulfonsäure zurückgewonnen.

2) 575 g (6 mol) Fluorbenzol, 101,5 g (0,5 mol) Terephthalsäuredichlorid und 75 g (0,5 mol) Trifluormethansulfonsäure wurden wie im Beispiel 1 umgesetzt. Die Ausbeute betrug nach 6 Rückführungen 93% d.Th., die Reinheit über 96% ; Fp : 218-220°C.

3) 575 g (6 mol) Fluorbenzol, 101,5 g (0,5 mol) Terephthalsäuredichlorid und 37,5 g (0,25 mol) Trifluormethansulfonsäure wurden wie im Beispiel 1 umgesetzt. Die Ausbeute betrug nach 7 Rückführungen 85,5% d.Th., die Reinheit über 96% ; Fp. 218-220°C.

4) 575 g (6 mol) Fluorbenzol, 101,5 g (0,5 mol) Terephthalsäuredichlorid und 18,8 g (0,125 mol) Trifluormethansulfonsäure wurden wie im Beispiel 1 umgesetzt. Die Ausbeute betrug nach 6 Rückführungen 81,3% d.Th., die Reinheit über 95% ; Fp. 218-220°C.

5) 575 g (6 mol) Fluorbenzol, 101,5 g (0,5 mol) Terephthalsäuredichlorid und 116 g (0,5 mol) 2-Hydroperfluorpropansulfonsäure wurden wie im Beispiel 1 umgesetzt. Die Ausbeute betrug nach 6 Rückführungen 94,6% d.Th., die Reinheit über 96% ; Fp. 218-220°C.

6) 575 g (6 mol) Fluorbenzol, 101,5 g (0,5 mol) Terephthalsäuredichlorid und 78 g (0,25 mol) 2-Hydroperfluorpropansulfonsäure wurden wie im Beispiel 1 umgesetzt. Die Ausbeute betrug nach 5 Rückführungen 91,7% d.Th., die Reinheit über 95% ; Fp. 218-220°C.

Bei der Weiterverarbeitung wie im Beispiel 1 erhielt man ein in Diphenylsulfon gelöstes BFB, das ohne weitere Reinigung zur Polykondensation verwendet werden konnte. Die Ausbeute betrug 89,3% (bezogen auf BFB), die Reinheit (ohne Lösungsmittel) über 98%.

7) 575 g (6 mol) Fluorbenzol, 101,5 g (0,5 mol) Terephthalsäuredichlorid und 99,3 g (0,5 mol) 2-Chlor-1,1,2-trifluoräthansulfonsäure wurden wie im Beispiel 1 umgesetzt. Die Ausbeute betrug nach 4 Rückführungen 96,6% d.Th., die Reinheit 95% ; Fp.218-220°C.

8) 575 g (6 mol) Fluorbenzol, 101,5 g (0,5 mol) Terephthalsäuredichlorid und 91 g (0,5 mol) 2-Hydroperfluoräthansulfonsäure wurden wie im Beispiel 1 umgesetzt. Die Ausbeute betrug nach 2 Rückführungen 95,7% d.Th., die Reinheit 96% ; Fp. 218-220°C.

9) 288 g (3 mol) Fluorbenzol, 31,5 g (0,15 mol) Terephthalsäuredichlorid und 40,1 g (0,1 mol) Perfluorhexansulfonsäure wurden wie im Beispiel 1 umgesetzt. Die Ausbeute betrug nach einer Rückführung 45% d.Th., die Reinheit ca. 80%. Der Rest bestand hauptsächlich aus Perfluorhexansulfonsäure.

10) 192 g (2 mol) Fluorbenzol, 20,3 (0,1 mol) Terephthalsäuredichlorid und 5 g (0,033 mol) Trifluormethansulfonsäure wurden 8 Stunden lang in einem 400 ml Autoklaven mit einer Auskleidung von Polytetrafluoräthylen auf 140°C erhitzt. Nach beendeter Reaktion wurde bis zum Erkalten auf Raumtemperatur gerührt, von der entstandenen Salzsäure entspannt und filtriert. Das Reaktionsprodukt wurde mit 20 g Fluorbenzol gewaschen ; die vereinte Mutterlauge wurde dann mit 20,3 g Terephthalsäuredichlorid wieder umgesetzt. Die Mutterlauge wurde danach in den bekannten Verfahrensablauf zurückgeführt. Die Ausbeute betrug nach 3 Rückführungen 95% d.Th., die Reinheit 95% ; Fp. 217-219°C.

11) 674 g (6 mol) Chlorbenzol, 101,5 g (0,5 mol) Terephthalsäuredichlorid und 75 g (0,5 mol) Trifluormethansulfonsäure wurden 20 h unter Rückfluß erhitzt. Nach dem Rühren bis zum Erkalten wurde das ausgefallene 1,4-Bis-(4-chlorbenzoyl)-benzol abgesaugt und mit 112 g (1 mol) Chlorbenzol gewaschen. Die Mutterlauge und das beim Waschen erhaltene Filtrat wurden vereinigt, mit 101,5 g Terephthalsäuredichlorid versetzt und weitere 20 h unter Rückfluß erhitzt. Die Mutterlauge wurde in der beschriebenen Weise fünfmal zurückgeführt. Die Ausbeute betrug 71% d.Th., die Reinheit ohne Chlorbenzol über 99% ; Fp. : 255-257°C.

**Vergleichsversuche**

V1) 575 g (6 mol) Fluorbenzol und 101,5 g (0,5 mol) Terephthalsäuredichlorid und 0,75 g (0,005 mol) Trifluormethansulfonsäure wurden 20 h unter Rückfluß erhitzt. Das Rohprodukt wurde abfiltriert und gewaschen.

Man erhielt BFB in einer Ausbeute von weniger als 10% ; die Reinheit betrug nur etwa 50%.

V2) 575 g (6 mol) Fluorbenzol, 101,5 g (0,5 mol) Terephthalsäuredichlorid und 7,5 g (0,05 mol) Trifluormethansulfonsäure wurden 20 h unter Rückfluß erhitzt. Nach dem Rühren bis zum Erkalten wurde abfiltriert und das Reaktionsprodukt mit 50 g Fluorbenzol gewaschen. Mutterlauge und Waschlösung wurden mit 101,5 g (0,5 mol) Terephthalsäuredichlorid versetzt und wie im Beispiel 1 umgesetzt. Die Ausbeute betrug nach 3 Rückführungen über 40%, die Reinheit ca. 60%.

## Patentansprüche

1. Verfahren zur Herstellung von halogenhaltigen aromatischen Verbindungen der Formel

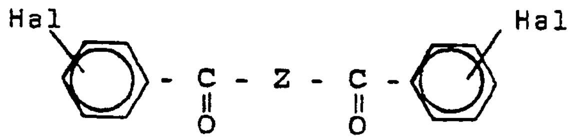

(I)

worin Hal Fluor, Chlor oder Brom und

Z eine aromatische Gruppierung bedeuten, dadurch gekennzeichnet, daß man ein Halogenbenzol der Formel $C_6H_5Hal$ (II) unter wasserfreien Bedingungen mit einem Bissäurehalogenid der Formel Hal-CO-Z-CO-Hal (III) im Molverhältnis von mindestens 2 : 1 in Gegenwart von 15 bis 500 mol-%, bezogen auf das Bissäurehalogenid (III), an Halogenalkansulfonsäuren der Formel $Y(C_nX_{2n})SO_3H$ (IV), umsetzt, wobei in den Formeln II und III Hal und Z die zuvor angegebene Bedeutung haben und Y Fluor oder Wasserstoff, X Fluor und/oder Chlor und n eine ganze Zahl von 1 bis 10 bedeuten, wobei mindestens ein X für Fluor steht.

2. Verfahren gemäß Anspruch 1, dadurch gekennzeichnet, daß nach Abtrennung der Verbindungen I die Mutterlauge — gegebenenfalls nach Ausschleusen eines kleinen Teils davon und Zugabe einer entsprechenden Menge Lösungsmittel und Katalysator — nach Zugabe von Bissäurehalogenid III und gegebenenfalls weiterem Halogenbenzol (II) wiederverwendet wird und daß vorzugsweise 6 bis 40, vorzugsweise 10 bis 30 Mol Halogenbenzol II mit je einem Mol der Verbindung (III) umgesetzt werden.

3. Verfahren gemäß Anspruch 1 oder 2, dadurch gekennzeichnet, daß Z Phenylen oder eine aromatische Gruppierung der Formel $C_6H_4$-E-$C_6H_4$ (VI) ist, worin E O,.$(CG_2)_m$, CO, S, SO, $SO_2$ oder $Si(CH_3)_2$, worin G Wasserstoff, Methyl, Fluor oder Trifluormethyl und m eine ganze Zahl von 0 bis 4 ist.

4. Verfahren gemäß einem oder mehreren der Ansprüche 1 bis 3, gekennzeichnet durch wenigstens eines der Merkmale, daß in den Verbindungen der Formeln (I) und (II) Hal Fluor oder Chlor und in Formeln (I) und (III) Z Phenylen, zweckmäßig in anderer als o-Stellung, insbesondere p-Phenylen ist und daß Halogen in einer anderen als der o-Stellung zu CO steht.

5. Verfahren gemäß einem oder mehreren der Ansprüche 1 bis 4, dadurch gekennzeichnet, daß Verbindungen der Formel (I) hergestellt werden, in denen das Halogen in p-Stellung zu CO steht.

6. Verfahren gemäß einem oder mehreren der Ansprüche 1 bis 5, dadurch gekennzeichnet, daß als Verbindungen (IV) solche der Formel $Y(C_nF_{2n})SO_3H$ (V) verwendet werden, worin Y Fluor oder Wasserstoff darstellt.

7. Verfahren gemäß einem oder mehreren der Ansprüche 1 bis 6, dadurch gekennzeichnet, daß bei der Verwendung von Verbindungen der Formel (IV) bzw. (V), in denen Y Wasserstoff ist, dieser in β-Stellung zur $SO_3H$-Gruppe steht.

8. Verfahren gemäß einem oder mehreren der Ansprüche 1 bis 7, dadurch gekennzeichnet, daß die Verbindung (IV) in einer Menge von 50 bis 150 mol-%, bezogen auf das Bissäurehalogenid (III), verwendet wird.

9. Verfahren gemäß einem oder mehreren der Ansprüche 1 bis 8, dadurch gekennzeichnet, daß man die Umsetzung der Verbindungen (II) und (III) bei einer Temperatur von 20 bis 200, vorzugsweise 60 bis 180°C durchführt.

10. Verfahren gemäß einem oder mehreren der Ansprüche 1 bis 9, dadurch gekennzeichnet, daß man die Umsetzung der Verbindungen II und III bei einem Druck von Atmosphärendruck bis zum Eigendruck des Reaktionsgemisches unter den angewandten Reaktionsbedingungen, vorzugsweise im Bereich von Atmosphärendruck bis zu 15 bar durchführt.

11. Verfahren gemäß einem oder mehreren der Ansprüche 1 bis 10, dadurch gekennzeichnet, daß man unter sauerstofffreien Bedingungen arbeitet.

12. Verwendung von Verbindungen der Formel I, vorzugsweise des 1,4-Bis-(4-fluorbenzoyl)-benzols und des 1,4-Bis-(4-chlorbenzoyl)-benzols, in Form einer klaren Lösung in Diphenylsulfon, die durch Behandlung

mit einer festen Base, vorzugsweise $Na_2CO_3$, $K_2CO_3$ oder MgO, erhalten worden ist, zur Herstellung von Polykondensaten.

## Claims

1. A process for the preparation of halogen-containing aromatic compounds of the formula

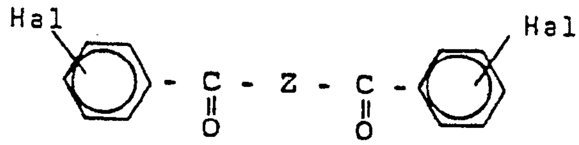

(I)

in which Hal denotes fluorine, chlorine or bromine and Z denotes an aromatic grouping, which comprises reacting a halobenzene of the formula $C_6H_5Hal$ (II) under anhydrous conditions with a bis(acid halide) of the formula Hal-CO-Z-CO-Hal (III) in a molar ratio of at least 2 : 1 in the presence of 15 to 500 mol%, relative to the bis (acid halide) (III), of haloalkanesulfonic acids of the formula $Y(C_nX_{2n})SO_3H$ (IV), where Hal and Z in the formulae II and III have the meaning previously indicated and Y denotes fluorine or hydrogen, X denotes fluorine and/or chlorine and n denotes an integer from 1 to 10, at least one X standing for fluorine.

2. The process as claimed in claim 1, wherein, after separating off the compounds I, the mother liquor — if desired after exclusion of a small part thereof and adding a corresponding amount of solvent and catalyst — is used again after addition of bis(acid halide) III and, if desired, further halobenzene (II) and wherein preferably 6 to 40, preferably 10 to 30, mol of halobenzene II are reacted with each mole of the compound (III).

3. The process as claimed in claim 1 or 2, wherein Z is phenylene or an aromatic grouping of the formula $C_6H_4$-E-$C_6H_4$ (VI), in which E is 0, $(CG_2)_m$, CO, S, SO, $SO_2$ or $Si(CH_3)_2$, in which G is hydrogen, methyl, fluorine or trifluoromethyl and m is an integer from 0 to 4.

4. The process as claimed in one or more of claims 1 to 3, having at least one of the features that in the compounds of the formulae (I) and (II) Hal is fluorine or chlorine and in the formulae (I) and (III) Z is phenylene, expediently in a position other than the o-position, in particular p-phenylene, and that halogen is in a position other than the o-position to CO.

5. The process as claimed in one or more of claims 1 to 4, wherein compounds of the formula (I) are prepared in which the halogen is in the p-position to CO.

6. The process as claimed in one or more of claims 1 to 5, wherein the compounds (IV) used are those of the formula $Y(C_nF_{2n})SO_3H$ (V) in which Y represents fluorine or hydrogen.

7. The process as claimed in one or more of claims 1 to 6, wherein, in the use of compounds of the formula (IV) or (V) in which Y is hydrogen, the latter is in the β-position to the $SO_3H$ group.

8. The process as claimed in one or more of claims 1 to 7, wherein the compound (IV) is used in an amount from 50 to 150 mol%, relative to the bis(acid halide) (III).

9. The process as claimed in one or more of claims 1 to 8, wherein the reaction of the compounds (II) and (III) is carried out at a temperature from 20 to 200, preferably 60 to 180°C.

10. The process as claimed in one or more of claims 1 to 9, wherein the reaction of the compounds II and III is carried out at a pressure from atmospheric pressure up to the intrinsic pressure of the reaction mixture under the reaction conditions used, preferably in the region of atmospheric pressure up to 15 bar.

11. The process as claimed in one or more of claims 1 to 10, wherein the reaction is carried out under oxygen-free conditions.

12. The use of compounds of the formula I, preferably of 1,4-bis(4-fluorobenzoyl)benzene and of 1,4-bis(4-chlorobenzoyl)benzene, in the form of a clear solution in diphenyl sulfone, which has been obtained by treatment with a solid base, preferably $Na_2CO_3$, $K_2CO_3$ or MgO, for the preparation of polycondensates.

## Revendications

1. Procédé de préparation de composés aromatiques halogénés de formule I

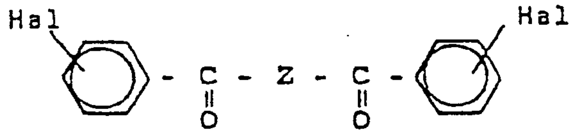

(I)

dans laquelle Hal désigne le fluor, le chlore ou le brome et Z un groupe aromatique, procédé caractérisé en ce que l'on fait réagir un halogénobenzène de formule $C_6H_5Hal$ (II), en milieu anhydre, avec un dihalogénure de diacide de formule Hal-CO-Z-CO-Hal (III) dans un rapport molaire d'au moins 2 :1, en présence de 15 à 500 mol-%, par rapport au dihalogénure de diacide (III), d'acides halogénoalcane-sulfoniques de formule $Y(C_nX_{2n})SO_3H$ (IV), dans les formules (II) et (III) Hal et Z ayant les significations ci-dessus indiquées et Y désignant le fluor ou l'hydrogène, X le fluor et/ou le chlore, et n un entier de 1 à 10, l'un au moins des radicaux X étant le fluor.

2. Procédé selon la revendication 1, caractérisé en ce que après avoir séparé les composés (I) on réutilise la liqueur-mère, éventuellement après en avoir éliminé une petite partie et lui avoir ajouté une quantité correspondante de solvant et de catalyseur, après addition du dihalogènure de diacide (III) et le cas échéant d'une nouvelle quantité de l'halogénobenzène (II), en faisant réagir de préférence de 6 à 40 mol de l'halogénobenzène (II), mieux encore de 10 à 30 mol, par mol du composé (III).

3. Procédé selon la revendication 1 ou 2, caractérisé en ce que Z est le groupe phénylène ou un groupe aromatique de formule $C_6H_4$-E-$C_6H_4$ (VI), E désignant O, $(CG_2)_m$, CO, S, SO, $SO_2$ ou $Si(CH_3)_2$, G l'hydrogène, le groupe méthyle, le fluor ou le groupe trichlorométhyle et m un entier de 0 à 4.

4. Procédé selon une ou plusieurs des revendications 1 à 3, caractérisé par une ou plusieurs des particularités suivantes : dans les composés de formules (I) et (II) Hal est le fluor ou le chlore et dans les formules (I) et (III) Z est le groupe phénylène, avantageusement non orthophénylène et en particulier p-phénylène, et l'halogène n'est pas à la position ortho par rapport au groupe CO.

5. Procédé selon une ou plusieurs des revendications 1 à 4, caractérisé en ce que l'on prépare des composés de formule (I) dont l'halogène est à la position para par rapport au groupe CO.

6. Procédé selon une ou plusieurs de revendications 1 à 5, caractérisé en ce que l'on utilise comme composés (IV) des composés de formule $Y(C_nF_{2n})SO_3H$ (V), Y étant le fluor ou l'hydrogène.

7. Procédé selon une ou plusieurs des revendications 1 à 6, caractérisé en ce que dans l'emploi de composés de formule (IV) ou (V) dans lesquels Y est l'hydrogène, celui-ci est à la position β par rapport au groupe $SO_3H$.

8. Procédé selon une ou plusieurs des revendications 1 à 7, caractérisé en ce que l'on utilise les composés (IV) dans des proportions de 50 à 150 mol-% par rapport au dihalogénure de diacide (III).

9. Procédé selon une ou plusieurs des revendications 1 à 8, caractérisé en que l'on effectue la réaction des composés (II) et (III) à une température de 20 à 200°C, de préférence de 60 à 180°C.

10. Procédé selon une ou plusieurs de revendications 1 à 9, caractérisé en ce que l'on effectue la réaction des composés (II) et (III) sous une pression comprise entre la pression atmosphérique et la pression autogène du mélange de réaction dans les conditions réactionnelles appliquées, de préférence entre la pression atmosphérique et 15 bar.

11. Procédé selon une ou plusieurs des revendications 1 à 10, caractérisé en ce que l'on opère à l'abri de l'oxygène.

12. L'emploi des composés de formule (I), de préférence du 1,4-bis(4-fluorobenzoyl)-benzène et du 1,4-bis(4-chlorobenzoyl)-benzène, en solution limpide dans de la biphénylsulfone, que l'on obtient avec une base solide, de préférence $Na_2CO_3$, $K_2CO_3$ ou MgO, pour la fabrication de produits de polycondensation.